# EUROPÄISCHE PATENTSCHRIFT

(11) **EP 2 775 362 B1**
(45) Veröffentlichungstag und Bekanntmachung des Hinweises auf die Patenterteilung: **25.03.2020**
(21) Anmeldenummer: 13004984.4
(22) Anmeldetag: 18.10.2013
(51) Int. Cl.: G05B 19/042

(54) **Feldgerät**
Field device
Appareil de terrain

(30) Priorität: 08.03.2013 DE 102013102327
(43) Veröffentlichungstag der Anmeldung: 10.09.2014
(73) Patentinhaber: Krohne Messtechnik GmbH, 47058 Duisburg (DE)
(72) Erfinder: van der Linde, Martin, 46147 Oberhausen (DE)
(74) Vertreter: Gesthuysen Patent- und Rechtsanwälte

(56) Entgegenhaltungen:
- WO-A1-02/37061
- WO-A1-2007/071211
- DE-A1-102006 062 603
- DE-A1-102010 028 152

## Beschreibung

Die Erfindung betrifft ein Feldgerät. Bei dem Feldgerät handelt es sich beispielsweise um ein Messgerät zur Bestimmung einer Mess- bzw. Prozessgröße oder um einen Aktor zur Einstellung bzw. Regelung einer Prozessgröße oder einer entsprechenden Umgebungsbedingung.

In der modernen Prozessautomatisierung werden mit unterschiedlichen Feldgeräten Prozesse überwacht oder gesteuert. Solche - als Messgeräte oder als Aktoren ausgestalteten - Feldgeräte sind dabei entweder als autarke Geräte ausgeführt oder sie sind beispielsweise über einen Feldbus mit einem Prozessleitsystem (PLS) verbunden. Für die Kommunikation mit einem solchen PLS bzw. mit einer von einem PLS umfassten Leitwarte dienen Kommunikationsbusse, beispielsweise Feldbusse gemäß dem Profibus- oder dem HART-Standard. Darüber hinaus lassen sich Feldgeräte noch dadurch unterscheiden, ob sie für die Datenkommunikation und die Stromversorgung eine gemeinsame Schnittstelle über zwei Leiter oder verschiedene Schnittstellen mit insgesamt vier (oder mehr) Leitern aufweisen. Entsprechend werden solche Geräte auch als Zwei- oder Vierleiter-Geräte bezeichnet.

Trotz aller Bemühungen und Optimierungen ist es nicht immer ausgeschlossen bzw. nicht ausschließbar, dass sich Fehler ereignen, dass unvorhergesehene Ereignisse eintreten oder dass Feldgeräte außerhalb ihrer Spezifikationen verwendet werden. Weiterhin ist es ggf. vorteilhaft oder erforderlich, dass Zustände protokolliert und damit auch dokumentiert werden. Darüber hinaus ist es für eine Verbesserung der Qualität von Feldgeräten vorteilhaft, wenn auch detaillierte Informationen über den dauernden Betrieb vorhanden sind.

Um an die in den Feldgeräten vorhandenen Daten zu gelangen, beschreibt beispielsweise die Patentschrift EP 0 964 325 B1 ein Verfahren, nach dem Status- oder Diagnosedaten aus Feldgeräten eingesammelt und an eine Auswerteeinheit übermittelt werden. Problematisch daran ist jedoch, dass die von den Feldgeräten bereitgestellte Datenmenge ggf. sehr hoch sein kann, so dass der Datenbus zwischen den Feldgeräten und einer Leitwarte sehr belastet werden kann. Dies ist insbesondere dann problematisch, wenn den Feldgeräten nur eine beschränkte Energie zur Verfügung steht. Zudem ist eine solche Methode bei nicht an einen Datenbus angeschlossenen Feldgeräten generell nicht möglich. Darüberhinaus ist es ggf. nicht erwünscht, dass alle Daten beispielsweise über den zu überwachenden bzw. zu steuernden Prozess über einen Feldbus übertragen werden.

Als Alternative beschreibt beispielweise die Offenlegungsschrift DE 10 2005 062 418 A1 einen separaten Datenlogger, der mit einem Feldgerät verbindbar ist und eine lokale Datenprotokollierung erlaubt. Nachteilig daran ist, dass dies eine mit dem Feldgerät zu verbindende zusätzliche Komponente darstellt.

DE 10 2010 028152 betrifft ein Feldgerät mit einer Historyfunktion zum protokollieren von Ein- und Ausschaltvorgängen und sonstigen Ereignissen in einem eigenen, dafür vorgesehenen Speichermodul im Feldgerät. Eine Speicherverwaltung wird nicht erwähnt.

Der Erfindung liegt daher die Aufgabe zugrunde, ein Feldgerät vorzuschlagen, das eine möglichst einfache Protokollierung von relevanten Betriebsdaten unter Vermeidung der Nachteile des Standes der Technik ermöglicht.

Das erfindungsgemäße Feldgerät, bei dem die zuvor hergeleitete und aufgezeigte Aufgabe gelöst ist, ist zunächst und im Wesentlichen dadurch gekennzeichnet, dass das Feldgerät mindestens eine als integraler Bestandteil des Feldgeräts ausgestaltete Datenspeichervorrichtung und mindestens eine als integraler Bestandteil des Feldgeräts ausgestaltete Steuereinheit aufweist. Dabei legt die Steuereinheit in Abhängigkeit von mindestens einem vorgebbaren Überwachungsereignis mindestens einen Datensatz in der Datenspeichervorrichtung ab.

Das erfindungsgemäße Feldgerät weist gleichsam einen eingebauten Datenlogger auf, der von einer dem Feldgerät innewohnenden Steuereinheit mit Daten versorgt wird. Damit nicht alle im Feldgerät erzeugten oder gewonnenen oder auftretenden Daten protokolliert werden, ist zumindest ein Überwachungsereignis vorgebbar, bei dessen Auftreten die Steuereinheit mindestens einen Datensatz passend zu diesem Überwachungsereignis in der Datenspeichervorrichtung ablegt, d. h. abspeichert. Die Steuereinheit kann dabei eine zusätzliche Komponente im Feldgerät sein, sie kann jedoch auch durch eine zentrale Intelligenz des Feldgerätes vertreten werden, also beispielsweise als Teil einer übergeordneten Recheneinheit realisiert sein. Die Daten, die mit dem jeweiligen Datensatz abzulegen sind, lassen sich in einer Ausgestaltung über einen Filter gezielt aussuchen oder werden alternativ beispielsweise bei der Fertigung des Feldgerätes fest vorgegeben.

Die folgenden Ausgestaltungen setzen sich vor allem mit den unterschiedlichen Überwachungsereignissen auseinander. Dabei kann jeweils ein einziges Überwachungsereignis zu einem Ablegen mindestens eines Datensatzes führen. Alternativ werden mehrere Überwachungsereignisse vorgegeben, deren Auftreten jeweils dazu führt, dass wenigstens ein Datensatz abgespeichert wird.

In einer Ausgestaltung besteht das Überwachungsereignis darin, dass ein Fehlerzustand im Feldgerät vorliegt. Hierfür ist es entsprechend erforderlich, dass im Feldgerät erkannt wird, dass ein Fehler besteht. Dies lässt sich beispielsweise durch den Ablauf von Diagnoseprogrammen realisieren oder dadurch, dass ungewöhnliche Werte, z. B. Spannungsabfälle oder Sprünge in der Stromstärke usw. erkannt werden. Die Selbstüberwachung von Feldgeräten ist beispielsweise beschrieben in der NAMUR-Empfehlung NE 107 mit dem Titel: "Selbstüberwachung und Diagnose von Feldgeräten".

In einer weiteren Ausgestaltung ist mindestens eine Messeinheit zum Ermitteln mindestens einer Messgröße vorgesehen. Dabei besteht das Überwachungsereignis darin, dass der von der Messeinheit ermittelte Wert der Messgröße außerhalb eines vorgebbaren Intervalls liegt. In dieser Ausgestaltung handelt es sich bei dem Feldgerät um ein Messgerät zur Bestimmung oder Überwachung einer Messgröße (beispielsweise ist dies der Füllstand, der Durchfluss, der pH-Wert, die Temperatur, die Viskosität, die elektrische Leitfähigkeit oder der Sauerstoffgehalt eines Mediums). Das zugehörige Überwachungsereignis ist dabei das Vorliegen eines Messwerts außerhalb eines Intervalls, z. B. das Überschreiten eines Grenzwerts. Ein solches Intervall kann beispielsweise durch die Spezifikation des Geräts oder der verwendeten Komponenten gegeben oder ggf. der der Bestimmung der Messgröße dienenden Algorithmen geschuldet sein.

In einer ergänzenden Ausgestaltung ist mindestens eine Sensoreinheit zum Ermitteln mindestens einer sekundären Messgröße vorgesehen. Hierfür besteht das Überwachungsereignis darin, dass der von der Sensoreinheit ermittelte Wert der sekundären Messgröße außerhalb eines vorgebbaren Intervalls liegt.

Teilweise hängt die eigentliche oder primäre Messgröße von anderen zusätzlichen Prozessgrößen ab. Bei der Bestimmung des Durchflusses eines Mediums oder dessen pH-Wert ist dies beispielsweise die Temperatur des Mediums. Weiterhin können auch die Messeinheiten für die primäre Messgröße selbst durch eine andere Prozessgröße beeinflusst werden, so dass eine diesbezügliche Messung erforderlich bzw. angezeigt ist. So erlauben beispielsweise Sensorkomponenten der Messeinheit nur eine sichere Anwendung innerhalb gewisser Rahmenbedingungen. Eine solche sekundäre Messgröße kann jedoch auch bei einem Feldgerät in Form eines Aktors darin bestehen, dass über diese Messgröße erfasst wird, ob die eingreifende Handlung des Aktors erfolgreich ist. Auch für diese sekundäre Messgröße wird wenigstens ein entsprechendes Intervall vorgegeben, dessen Überschreiten ein Überwachungsereignis darstellt, das durch die Steuereinheit erfindungsgemäß in der Datenspeichervorrichtung mit einem Datensatz protokolliert wird.

Ist in einer Ausgestaltung die Messeinheit zur Ermitteln des Füllstands, des Durchflusses oder des pH-Werts als Messgröße ausgebildet, so sieht es eine weitere alternative oder ergänzende Ausgestaltung vor, dass die Sensoreinheit zur Ermittlung der Temperatur oder des Drucks als sekundärer Messgröße dient. Weitere Messgrößen und eine entsprechende Ausgestaltung der Messeinheit bzw. der Sensoreinheit liegen dabei nahe und lassen sich durch Komponenten des Standes der Technik realisieren.

In einer weiteren Ausgestaltung verfügt das Feldgerät über mindestens eine Zugriffsschnittstelle zur Ermöglichung eines Zugriffs auf das Feldgerät. Dabei handelt es sich beispielsweise um eine sogenannte Service-Schnittstelle, die beispielsweise auch das Einspielen von Soft- oder Firmware erlaubt und über die insbesondere auch die Übermittlung von größeren Datenmengen, z. B. zum Abfragen der Datensätze der Datenspeichervorrichtung möglich ist.

Alternativ handelt es sich um eine Benutzerschnittstelle in Form eines sogenannten Man-Machine-Interface, über dessen Display und eine Realisierung einer Eingabemöglichkeit ein Zugriff auf das Feldgerät möglich ist. So lassen sich beispielsweise aus einer Reihe von vorgegebenen Parametern einzelne Parameter entsprechend für die Anwendung auswählen oder modifizieren.

In diesem Zusammenhang ist bei einer solchen Zugriffsschnittstelle als ein mögliches Überwachungsereignis vorgesehen, dass über die Zugriffsschnittstelle - generell - ein Zugriff und/oder - speziell und damit ausgesucht - eine bestimmte Art von Zugriff auf das Feldgerät stattfindet. In dieser Variante wird also ein Einwirken auf das Feldgerät protokolliert.

In einer besonderen Ausgestaltung, in der eine spezielle Art des Einwirkens auf das Feldgerät als Überwachungsereignis vorgegeben ist, legt die Steuereinheit in dem Fall, dass über die Zugriffsschnittstelle mindestens ein Bedienparameter des Feldgeräts geändert wird, als Datensatz mindestens Daten über den Zeitpunkt der Änderung oder Daten über den Bedienparameter oder einen vor der Änderung gegebenen Wert des Bedienparameters oder Daten über die den Bedienparameter ändernde Instanz in der Datenspeichervorrichtung ab.

Der Datensatz, der für das Eintreten des Überwachungsereignisses "Änderung mindestens eines Parameters" in der Datenspeichervorrichtung abgelegt wird, umfasst also wenigstens eine Art von Zeitstempel, eine Angabe über den geänderten Parameter, den alten und/oder neuen Wert oder eine Identifizierungsmöglichkeit für die Person oder allgemein Instanz, die die Änderung vorgenommen hat. Ein Zeitstempel kann dabei ausgehen von einer im Feldgerät befindlichen Uhr oder von Zeitangaben, die beispielsweise über den Busanschluss dem Feldgerät übermittelt werden. Der geänderte Parameter kann insbesondere über eine Kodierung im Datensatz abgelegt werden. Entsprechend lassen sich auch die alten bzw. neuen Werte durch absolute oder relative Angaben abspeichern. Eine Angabe über die ändernde Instanz ist insbesondere dann relevant, wenn eine Parameteränderung nicht für einen beliebigen Personenkreis möglich ist, sondern einzelnen Personen bzw. Personenklassen vorbehalten ist. Ist also insbesondere ein Zugangscode erforderlich, so kann beispielweise dieser oder die damit einhergehende Berechtigung in dem dem Überwachungsereignis zugeordneten Datensatz abgelegt werden.

Die Daten als Teile des in der Datenspeichervorrichtung abzulegenden bzw. abzuspeichernden Datensatzes bzw. der Datensätze werden in der folgenden Ausgestaltung näher angegeben.

Die Steuereinheit legt als Datensatz mehrere vorgebbare Daten des Feldgeräts ab, wobei es sich bei den Daten mindestens um einen dem Überwachungsereignis zugeordneten Wert oder um eine Angabe eines Zeitpunkts oder um einen einer Abfolge eines Auftretens des Überwachungsereignisses zugeordneten Wert oder um eine Angabe über eine Betriebsdauer des Feldgeräts oder um mindestens einen vom Feldgerät ermittelten Wert für eine Messgröße oder um mindestens einen innerhalb des Feldgeräts erzeugten und/oder berechneten Wert handelt. Die Daten lassen sich in einer Ausgestaltung durch einen Benutzer auswählen und werden in einer alternativen Ausführung in Abhängigkeit vom für die Speicherung zur Verfügung stehenden Speicherplatz der Datenspeichervorrichtung und/oder der für die Speicherung zur Verfügung stehenden Energie optimiert ausgewählt.

Die Daten des Datensatzes charakterisieren einerseits das Überwachungsereignis in Bezug auf eine Kodierung des jeweiligen Überwachungsereignisses oder dessen Auftreten. So wird beispielsweise ein Zähler für die Häufigkeit des Ereignisses mitgeführt oder es wird protokolliert, ob das Überwachungsereignis nach einer Initialisierung oder im laufenden Betrieb auftrat.

Andererseits sind die Daten eine Momentaufnahme, also ein "Snapshot" der Bedingungen des Feldgeräts bzw. des Prozesses, insofern auch andere Messgrößen, aktuell berechnete oder ermittelte Werte, Zwischenwerte usw. abgelegt werden. Dazu kommt ggf. noch eine Angabe über die Betriebszeit des Feldgerätes bzw. welche Messgrößen oder Prozessparameter vor dem Eintreten des Überwachungsereignisses vorlagen.

Darüberhinaus kann es auch erforderlich bzw. hilfreich sein, wenn der Datensatz oder die mit dem Überwachungsereignis verbundenen Datensätze sich auf nach dem auslösenden Moment auftretende oder diesem vorausgehende Daten oder Messwerte usw. beziehen. Stellt das Überwachungsereignis beispielsweise einen außergewöhnlichen Messwert dar, so kann es beispielsweise vorteilhaft sein, anhand der in der Datenspeichervorrichtung abgelegten Datensätze zu verfolgen, welche Messgenauigkeit sich anschließend erzielen lässt. Alternativ kann bei einem Überschreiten eines Intervalls für die sekundäre Messgröße durch die Protokollierung der Werte der primären Messgröße die Auswirkung und insbesondere die Nachwirkung auf die eigentliche Messung identifiziert werden.

In einer weiteren Ausgestaltung wird nach einer Änderung eines Messparameters durch die Protokollierung der ermittelten Werte der primären Messgröße eine Auswirkung der Parameteränderung auf die Messungen erkenn- und vor allem auch belegbar.

In einer Ausgestaltung ist insbesondere vorgesehen, dass die Steuereinheit nach einem Auftreten des Überwachungsereignisses eine vorgebbare Anzahl von zusätzlichen Datensätzen in Verbindung mit dem Auftreten des Überwachungsereignisses in der Datenspeichervorrichtung ablegt. Diese Datensätze erlauben es bei einer Auswertung, die Auswirkung des Überwachungsereignisses auf das Feldgerät bzw. auf dessen Daten oder allgemein sogar auf den Prozess, den das Feldgerät misst oder in den es eingreift, zu beobachten.

Erstreckt sich das Überwachungsereignis über einen gewissen Zeitraum, so ist in einer Ausgestaltung die Steuereinheit derartig ausgerichtet, dass sie in dem Fall mindestens einen Datensatz in der Datenspeichervorrichtung ablegt, wenn das Überwachungsereignis beendet ist. In dieser Ausgestaltung wird also eine Momentaufnahme nach dem Ende des Überwachungsereignisses protokolliert.

Alternativ oder ergänzend legt die Steuereinheit in einer weiteren Ausgestaltung bei einem zeitlich anhaltenden Überwachungsereignis mindestens die zeitliche Dauer des Bestehens bzw. Vorliegens des Überwachungsereignisses als Datensatz und/oder als Teil eines Datensatzes in der Datenspeichervorrichtung ab. Hierbei wird zumindest die Dauer des Überwachungsereignisses gespeichert.

Wiederholt sich ein Überwachungsereignis, so legt die Steuereinheit in einer Ausgestaltung bei einem wiederholten Auftreten die Anzahl des Auftretens des Überwachungsereignisses als Datensatz und/oder als Teil eines Datensatzes in der Datenspeichervorrichtung ab. Von der Steuereinheit wird also in dieser Ausgestaltung mitgeschrieben, wie oft das Ereignis stattfindet. Dabei lassen sich auch je nach Art der Überwachungsereignisse die unterschiedlichen Ausgestaltungen entsprechend miteinander kombinieren.

Dieses Mitlaufenlassen eines Zählers ist insbesondere dann vorteilhaft, wenn aufgrund des zur Verfügung stehenden Speicherplatzes oder für die Reduzierung der abgelegten Datensätze insbesondere für eine Auswertung nicht für jedes Auftreten des Überwachungsereignisses die Datensätze in der Datenspeichervorrichtung vorgehalten, sondern teilweise gelöscht oder überschrieben werden.

So besteht eine Ausgestaltung darin, dass die Steuereinheit bei einem wiederholten Auftreten eines Überwachungsereignisses in dem Fall, dass das Überwachungsereignis auftritt, in der Datenspeichervorrichtung mindestens einen für ein vorhergehendes Auftreten des Überwachungsereignisses abgelegten Datensatz überschreibt oder löscht. In dieser Ausgestaltung werden also nicht alle zur gleichen Art von Überwachungsereignis zugehörigen und vor dem aktuellen Auftreten abgelegten Datensätze aufbewahrt, sondern wenigstens ein vorhergehender und dadurch alter Datensatz wird gelöscht oder durch den jeweiligen neuen Datensatz überschrieben.

Dabei kann beispielsweise das erste Auftreten des Überwachungsereignisses bzw. dessen zugeordneter Datensatz vom Überschreiben ausgeklammert werden, so dass dieser Datensatz erhalten bleibt. Auch können weitere Kriterien gesetzt werden, die ein Sicherung eines Datensatzes gewährleisten.

In einer Ausgestaltung wird die Steuereinheit in einer sogenannten Einlernphase mit den Bedingungen vertraut gemacht, die das Vorliegen eines Überwachungsereignisses kennzeichnen. Dies erfolgt beispielsweise über eine Eingabe der spezifischen, zu beachtenden Grenzwerte oder dadurch, dass die Bedingungen, die beispielsweise als Messgröße dem Feldgerät zugänglich sind, angefahren werden, während das Feldgerät in dieser Einlernphase misst oder als Aktor auf einen Prozess einwirkt.

In einer Ausgestaltung ist vorgesehen, dass die Steuereinheit eine Anzahl und/oder eine Größe der Daten des Datensatzes in Abhängigkeit von einer Speichergröße der Datenspeichervorrichtung und/oder einer für eine Speicherung des Datensatzes zur Verfügung stehenden Speichergröße der Datenspeichervorrichtung und/oder einer für eine Speicherung des Datensatzes zur Verfügung stehenden Energie festsetzt. Da die Datenspeichervorrichtung ein Teil des Feldgerätes ist und daher entsprechenden Beschränkungen unterliegt, skaliert die Steuereinheit in dieser Ausgestaltung die Anzahl der abzulegenden Datensätze bzw. deren Größe auf den jeweils verfügbaren Datenspeicher. Weiterhin kann die Anzahl oder Größe der Daten von der Energie abhängen, die generell dem Feldgerät oder speziell für das Abspeichern der Daten zur Verfügung steht.

Sind insbesondere mehrere Überwachungsereignisse zu protokollieren, so muss unter Umständen auf den zur Verfügung stehenden Speicherplatz in der Datenspeichervorrichtung Rücksicht genommen werden. Weiterhin kann der Fall auftreten, das zwei Überwachungsereignisse zeitgleich oder in zeitlicher Nähe auftreten. Dafür ist es in einer Ausgestaltung vorgesehen, dass die unterschiedlichen Überwachungsereignisse mit unterschiedlichen Bedeutungsstufen, also mit unterschiedlichen Prioritätswerten versehen werden. Die Überwachungsereignisse werden gleichsam mit einer unterschiedlichen Wertigkeit versehen. Ausgehend von diesen Bedeutungsstufen oder den unterschiedlichen Prioritäten legt dann die Steuereinheit die mit den Überwachungsereignissen verbundenen Datensätze in der Datenspeichervorrichtung ab. So kann beispielweise einem Überwachungsereignis mit einer höheren Priorität auch ein größerer Speicherplatz für mehr abzulegende Daten zugewiesen werden als einem Überwachungsereignis mit niederer Priorität.

Bei der Vorgabe von mehreren unterschiedlichen Überwachungsereignissen ist es in einer Ausgabe auch vorgesehen, dass zu mindestens zwei unterschiedlichen Überwachungsereignissen zwei unterschiedlich aufgebaute Typen von Datensätzen abgelegt werden. Die Datensätze unterscheiden sich dabei insbesondere in Hinblick auf die Struktur der Daten, so z. B. die Abfolge der einzelnen Daten oder deren Größe in Bezug auf Minimal- und/oder Maximalgröße. In dieser Ausgestaltung werden also die Datensätze auf das zugeordnete Überwachungsereignis passend zugeschnitten bzw. optimiert. In einer weiteren Ausgestaltung ist für jedes Überwachungsereignis ein spezieller Typ von Datensatz vorgesehen.

Umgekehrt lässt sich die Datenablage und auch das Auslesen bzw. Auswerten der Daten aus der Datenspeichervorrichtung in dem Fall, dass mehrere Überwachungsereignisse definiert sind, dadurch vereinfachen, dass die Steuereinheit zu allen Überwachungsereignissen die Datensätze als gleicher Typ eines Datensatzes in der Datenspeichervorrichtung ablegt. Für alle bzw. zumindest für alle ausgewählten, sozusagen aktivierten Überwachungsereignisse wird in dieser Ausgestaltung jeweils mindestens ein Datensatz der gleichen Ausbildung bzw. der gleichen Struktur abgelegt.

Da sich die Überwachungsereignisse hinsichtlich der jeweils zur Verfugung stehenden bzw. für die Beurteilung relevanten Daten voneinander unterscheiden können, ist in einer Ausgestaltung vorgesehen, dass die Steuereinheit mindestens einen Teil der Daten eines Datensatzes, der bei einem Überwachungsereignis frei von einem Wert ist, durch mindestens einen Platzhalter beim Ablegen des Datensatzes auffüllt. Dadurch ergeben sich zumindest stets uniforme Datensätze, die einer Auswertung oder einer Weiterverarbeitung leichter zugänglich sind.

Da die Speicherkapazität bei einer Datenspeichervorrichtung technisch bedingt beschränkt ist, verwendet in einer Ausgestaltung die Steuereinheit die Datenspeichervorrichtung in Art eines Ringspeichers, so dass die ältesten Datensätze überschrieben werden. Dabei werden in einer Ausgestaltung einzelne Datensätze oder zumindest ein Datensatz gezielt vor einem Überschreiben bzw. einem Löschen geschützt.

Für die Speicherung bzw. das Ablegen der Datensätze richtet sich in einer Ausgestaltung die Steuereinheit nach einer vorgebbaren Datensatzstruktur, die den zur Verfügung stehenden Speicherplatz der Datenspeichervorrichtung aufteilt oder einzelne Bereiche definiert.

Alternativ legt die Steuereinheit die Datensätze aufeinander folgend ab oder die Daten der Datensätze werden in unzusammenhängenden Bereichen der Datenspeichervorrichtung ablegt.

Um die Datensätze und ggf. einzelnen Daten aus der Datenspeichervorrichtung auszulesen - als Alternative oder als Ergänzung zu einer aus dem Feldgerät herausnehmbaren Datenspeichervorrichtung - verfügt das Feldgerät in einer Ausgestaltung mindestens über eine Datenschnittstelle für einen lesenden Zugriff auf die Datenspeichervorrichtung.

Bei der Datenschnittstelle handelt es sich in einer Ausgestaltung um diejenige, über welche das Feldgerät mit einer Leitwarte oder einem Feldbus verbindbar ist. Dabei kommuniziert in einer Ausgestaltung eine Zentraleinheit des Feldgeräts mit der Datenschnittstelle und greift - ggf. über die Steuereinheit - auf die Daten der Datenspeichervorrichtung zu.

Für einen solchen Zugriff über eine solche Datenschnittstelle oder auch für das Auslesen der Daten bei einer aus dem Feldgerät herausgenommenen Datenspeichervorrichtung ist es in einer Ausgestaltung vorgesehen, dass nicht auf alle Daten bzw. Datensätze frei zugegriffen werden kann, sondern dass mindestens ein abgelegter Datensatz oder ein Teil eines abgelegten Datensatzes nur nach einer Freigabeaktion aus der Datenspeichervorrichtung auslesbar ist. Diese Freigabeaktion besteht beispielsweise darin, dass ein entsprechender Freigabecode eingegeben wird.

Mit einer Datenausleseprozedur ist eine Ausgestaltung verbunden, die darin besteht, dass die Steuereinheit bei einem lesenden Zugriff auf die Datenspeichervorrichtung das Auftreten des lesenden Zugriffs als Datensatz und/oder als Teil eines Datensatzes in der Datenspeichervorrichtung ablegt. So wird beispielweise bei dem ausgelesenen Datensatz ein Zeichen für das Auslesen gesetzt.

In einer Ausgestaltung ist die Datenspeichervorrichtung oder wenigstens ein Teil von ihr aus dem Feldgerät entnehmbar und entsprechend wieder einführbar. Handelt es sich beispielweise um eine Speicherkarte, so wird diese aus dem Feldgerät herausgezogen, die Daten werden über einen Computer extrahiert und anschließend wird sie wieder in die Datenspeichervorrichtung eingeführt.

Um eine Sicherung der abgelegten Datensätze zu erreichen, ist in einer Ausgestaltung die Datenspeichervorrichtung innerhalb des Feldgeräts gekapselt, also gegenüber Einwirkungen, die sich auf das Feldgerät auswirken, noch einmal separat geschützt. Vorzugsweise ist die Kapselung derartig, dass ein Zugriff auf die abgelegten Datensätze auch dann noch möglich ist, wenn ein Teil des Feldgerätes zerstört ist.

Die obigen Ausführungen und Ausgestaltung bezogen sich teilweise verstärkt auf ein Feldgerät, das als Messgerät ausgestaltet ist, um eine Messgröße zu bestimmen bzw. zu überwachen. Dabei beziehen sich die obigen Ausgestaltungen jedoch auch auf ein Feldgerät, das als Aktor ausgeformt ist, um damit mindestens eine Prozessgröße zu beeinflussen. Daher ist in einer Ausgestaltung mindestens eine Eingriffseinheit zum Einstellen mindestens einer Prozessgröße vorgesehen. Bei der Eingriffseinheit handelt es sich beispielsweise um ein Ventil, um eine Heiß- oder Kühlvorrichtung oder um eine Schranke usw.

In einer Ausgestaltung ist das Feldgerät als Zweileiter-Gerät ausgestaltet, so dass die Energieversorgung und die Datenübermittlung über den gleichen Anschluss erfolgt. Alternativ ist das Feldgerät als Vierleiter-Gerät ausgeführt. In einer weiteren Variante handelt es sich um ein autarkes Gerät, das mit einer Energiespeichereinheit und in einer weiteren Ausgestaltung zusätzlich mit einer Vorrichtung für das "Energy Harvesting" versehen ist.

Im Einzelnen gibt es eine Vielzahl von Möglichkeiten, das erfindungsgemäße Feldgerät auszugestalten und weiterzubilden. Dazu wird verwiesen einerseits auf die dem Patentanspruch 1 nachgeordneten Patentansprüche, andererseits auf die folgende Beschreibung von Ausführungsbeispielen in Verbindung mit der Zeichnung. In der Zeichnung zeigen
- Fig. 1: eine schematische, im Wesentlichen die funktionalen Wirkzusammenhänge verdeutlichende und teilweise als Blockschaltbild ausgeführte Darstellung eines erfindungsgemäßen Feldgeräts in einer ersten Ausführung,
- Fig. 2: eine schematische Darstellung eines erfindungsgemäßen Feldgeräts in einer zweiten Ausführung,
- Fig. 3: eine schematische Darstellung eines Teils eines erfindungsgemäßen Feldgeräts,
- Fig. 4: eine schematische Darstellung der Bestandteile eines Datensatzes und
- Fig. 5: ein schematischer Ablauf für die zeitliche Abfolge des Auftretens einer Überwachungsereignisses.

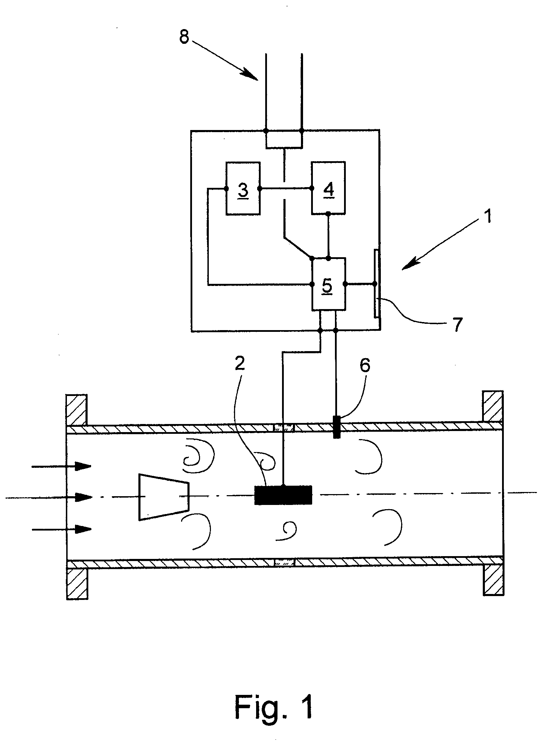

In der Fig. 1 ist ein erstes Ausführungsbeispiel eines erfindungsgemäßen Feldgeräts 1 dargestellt.

In dieser Variante handelt es sich bei dem Feldgerät 1 um ein Messgerät zur Bestimmung einer Messgröße oder zum Überwachen dieser Messgröße. Für die Messung ist dabei eine Messeinheit 2 vorgesehen. Die Messeinheit 2 bestimmt in der hier dargestellten Ausgestaltung insbesondere Druckschwankungen, die durch Wirbel in einem strömenden Medium (angedeutet durch die Pfeile) auftreten.

Die Durchflussmessung erfolgt insgesamt nach dem Vortex-Prinzip. Dieses Messprinzip beruht darauf, dass sich in dem fließfähigen Medium hinter einem Staukörper, der von dem Medium in einem Messrohr umströmt wird, eine sogenannte Kármánsche Wirbelstraße ausbilden kann, die durch sich mit der Strömung fortbewegende und sich von dem Staukörper ablösende Wirbel gebildet ist. Die Frequenz, mit der sich die Wirbel ablösen, ist von der Strömungsgeschwindigkeit abhängig, wobei der Zusammenhang unter gewissen Bedingungen nahezu linear ist. Über die Messung der Wirbelfrequenz mit einer Messeinheit 2 kann daher die Strömungsgeschwindigkeit des Mediums bestimmt werden.

In dem Feldgerät 1 als integrale Bestandteile sind eine Datenspeichervorrichtung 3 und eine Steuereinheit 4 vorgesehen. Integral bedeutet dabei, dass diese Komponenten im Feldgerät 1 befindliche und zu dessen Vollständigkeit gehörende Teile sind. Sie sind daher nicht beispielsweise zusätzlich hinzugeschaltet.

Die Steuereinheit 4 greift zumindest schreibend bzw. datenablegend auf die Datenspeichervorrichtung 3 zu, so dass entsprechende Verbindungen vorgesehen sind.

Für das Arbeiten des Feldgeräts 1 und insbesondere für die Durchführung der Messungen über die Messeinheit 2 ist eine Zentraleinheit 5 als primäre Steuereinheit des Feldgeräts 1 vorgesehen. In einer alternativen - nicht dargestellten - Ausgestaltung ist die Steuereinheit 4 ein Teil der Zentraleinheit 5 bzw. übernimmt die Zentraleinheit 5 die Aufgaben der Steuereinheit 4. Von der Zentraleinheit 5 bekommt die Steuereinheit 4 auch die Daten bzw. greift die Steuereinheit 4 die Daten ab, die sie beim Vorliegen des Überwachungsereignisses oder eines der vorbestimmten Überwachungsereignisse in der Datenspeichervorrichtung 3 ablegt.

Die Steuereinheit 4 legt in Abhängigkeit von vorgebbaren Überwachungsereignissen Datensätze in der Datenspeichervorrichtung 3 ab. Dieses Ablegen bzw. Abspeichern ist dabei ein rein feldgerät-interner Vorgang, der vom Feldgerät 1 selbst mit integralen Bestandteilen des Feldgeräts 1 vorgenommen wird. Um welche Daten - insbesondere eine Kodierung des Überwachungsereignisses, ein Zeitstempel, eine Momentaufnahme von im Feldgerät 1 vorliegenden Daten usw. - es sich handelt, wird im Folgenden noch verdeutlicht. Die Daten werden dabei ggf. je nach Anwendung oder Art des Überwachungsereignisses spezifisch ausgewählt bzw. aus der Menge alle möglichen im Feldgerät vorliegenden Daten herausgefiltert.

In einer Variante gibt es eine Mehrzahl von möglichen Überwachungsereignissen, die aus einer vorgebbaren Liste ausgewählt oder individuell für die Art des Feldgeräts 1 oder die Art der Anwendung definiert werden. Ein Überwachungsereignis kann dabei durch eine Bedingung gekennzeichnet sein, so z. B. durch das Auftreten eines Messwerts oder das Auslösen eines Vorganges. Alternativ kann es sich aber auch um einen komplexen Zustand handeln, der durch das Auftreten mehrerer Bedingungen gekennzeichnet ist.

Mögliche Überwachungsereignisse sind beispielsweise das Vorliegen eines Fehlerzustands im Feldgerät 1, ein Datenzugriff auf das Feldgerät 1, das Auftreten eines Messwerts für die mit der Messeinheit 2 zu messende Messgröße außerhalb eines vorgegebenen Intervalls oder das Vorliegen eines Messwerts, der mit einer Sensoreinheit 6 für eine sekundäre Messgröße ermittelt wird, außerhalb eines spezifizierten Wertebereichs.

Die Sensoreinheit 6 dient hier beispielsweise der Temperaturmessung, so dass die Steuereinheit 4 in der Datenspeichervorrichtung 3 einen Datensatz ablegt, wenn die Temperatur des Mediums außerhalb eines vorgegebenen und für die Anwendung des Feldgeräts 1 spezifizierten Temperaturintervalls liegt. Ein solches Intervall kann dabei ggf. auch nur durch einen Grenzwert hinreichend beschrieben sein.

Alternativ oder ergänzend werden in Abhängigkeit von der Betriebsdauer des Feldgeräts 1 Datensätze erzeugt und abgelegt oder werden Daten über die Betriebsdauer innerhalb von mit entsprechenden Überwachungsereignissen verknüpften Datensätzen abgelegt.

In einer Ausgestaltung einer solchen erweiterten Betriebsdauererfassung werden in Abhängigkeit von den Werten der mit der Messeinheit 2 gemessenen Messgröße - also hier des Durchflusses - die Datensätze abgelegt. Damit wird beispielweise eine Art erweiterte Betriebszeiterfassung vorgenommen, indem mitgeschrieben wird, wie oft bzw. über welchen Zeitraum hinweg die Messgröße sich innerhalb vorgegebener Intervalle befand. Die einzelnen vorgegebenen Intervalle liegen dabei beispielsweise teilweise ineinander oder grenzen aneinander an oder sind ggf. teilweise auch überlappend.

Mit einer solchen Feinstruktur des Ablaufs der Messgrößen oder Prozessbedingungen ist eine Betrachtung der Belastung, der das Feldgerät 1 unterliegt, möglich bzw. kann beurteilt werden, ob das Feldgerät 1 innerhalb für die Benutzung vorgegebener Spezifikationen betrieben worden ist. Für diese letztere Betrachtung dient auch die Bestimmung der sekundären Messgröße über die Sensoreinheit 6 als Maß für die Prozess- bzw. Messbedingungen.

Ein weiteres mögliches Überwachungsereignis ist, dass über eine Zugriffsschnittstelle 7 ein Zugriff auf das Feldgerät 1 stattfindet.

Bei der Zugriffsschnittstelle 7 handelt es sich in einer Ausgestaltung um eine sogenannte Service-Schnittstelle, über die Daten ein- und ausgegeben werden können. In einer alternativen Ausgestaltung handelt es sich um eine Benutzerschnittstelle (ein sogenanntes Human-Machine-Interface, HMI) mit einem Display und dort visualisierten Tasten (z. B. eine Nachbildung einer klassischen Tastatur oder Auswahltasten wie "rechts"/"links", "oben"/"unten" und "bestätigen"). In einer weiteren - nicht dargestellten - Ausgestaltung handelt es sich bei der Zugriffsschnittstelle 7 um eine Feldbusschnittstelle, z. B. für die Kommunikation mit einer Prozessleitwarte.

Findet über eine solche Zugriffsschnittstelle 7 ein Zugriff auf bzw. Eingriff in den Ablauf des Feldgeräts 1 statt, so kann dies - je nach Auswahl - als das Auftreten eines zu überwachenden Überwachungsereignisses gewertet werden, wofür ein entsprechender Datensatz in der Datenspeichervorrichtung 3 abzulegen ist.

Hierbei wird entweder jeder Zugriff durch das Ablegen eines Datensatzes protokolliert oder es findet eine Beschränkung auf spezielle Zugriffe statt, wie z. B. das Ändern von Betriebs- oder Messparametern des Feldgeräts 1. Die relevanten Daten bei einer solchen Parameteränderung sind z. B. der geänderte Parameter oder ein Code für diesen, der vorherige Wert des Parameters oder ggf. zudem der Wert, auf den der Parameter gesetzt worden ist. Ist eine solche Parameteränderung zudem nur einzelnen Personen oder Personengruppen vorbehalten und müssen sich diese vor einer Änderung identifizieren, so wird vorzugsweise ebenfalls diese Zuordnung im entsprechenden Datensatz abgelegt.

Weiterhin verfügt das Feldgerät 1 in der dargestellten Variante über eine Datenschnittstelle 8, die hier eine Zweileiter-Schnittstelle ist und daher auch der Energieversorgung des Feldgeräts 1 dient.

Über diese Datenschnittstelle 8 lassen sich - im gezeigten Fall vermittelt durch die Zentraleinheit 5 - Daten bzw. Datensätze aus der Datenspeichervorrichtung 3 auslesen. Dabei ist der Zugriff ggf. auf gewisse Datensätze, die z. B. besonderen Überwachungsereignissen zugeordnet sind, beschränkt bzw. sind nicht für jeden Zugriff alle Daten oder Datensätze zugänglich. Daher ist es in einer Ausprägung erforderlich, dass eine zu spezifizierende Freigabeaktion stattfindet. Dies besteht in einer Ausgestaltung darin, dass ein Freischaltcode an das Feldgerät 1 - vorzugsweise über die Zugriffsschnittstelle 7 oder über die Datenschnittstelle 8 selbst - übermittelt wird.

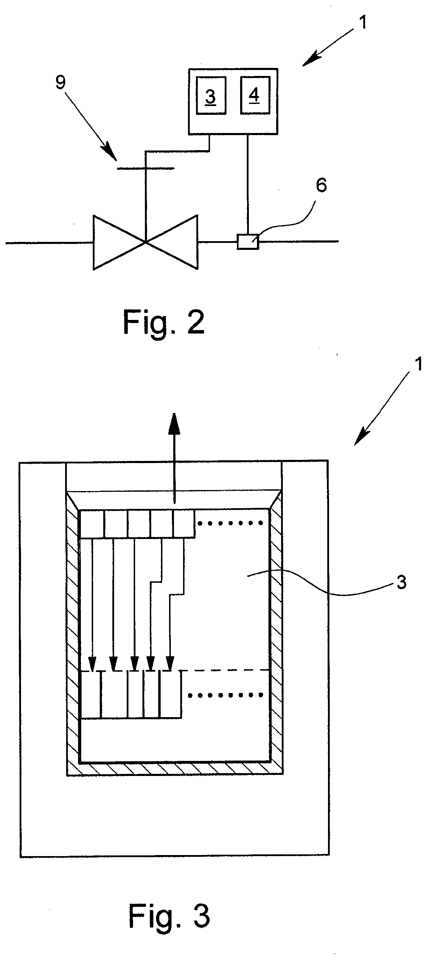

Die Fig. 2 zeigt ein erfindungsgemäßes Feldgerät 1, das als Aktor ausgestaltet ist. Dabei ist ein Ventil als Eingriffseinheit 9 für den Durchfluss eines Mediums durch eine angedeutete Leitung vorgesehen. Als Sensoreinheit 6 zur Bestimmung bzw. Überwachung einer sekundären - oder vielmehr, da diese Variante des Feldgeräts 1 ohne primäre Messgröße auskommt, direkt einzigen - Messgröße dient in dieser Ausgestaltung ein Druckmessgerät. Auch bei diesem Feldgerät 1 sind eine Datenspeichervorrichtung 3 und eine Steuereinheit 4 für die Datenablage innerhalb der Datenspeichervorrichtung 3 vorgesehen.

Die Fig. 3 zeigt rein schematisch einen Teil eines erfindungsgemäßen Feldgeräts 1. Die Datenspeichervorrichtung 3 ist dabei insbesondere gegenüber einer Beschädigung innerhalb des Feldgerätes 1 gekapselt und lässt sich reversibel aus dem Feldgerät 1 entnehmen (angedeutet durch den Pfeil nach oben) und dann auch wieder einfügen.

Dargestellt sind weiterhin rein symbolisch fünf Datensätze, die jeweils infolge des Auftretens von unterschiedlichen Überwachungsereignissen abgelegt worden sind. Die Datensätze setzen sich aus jeweils zwei Teilen zusammen, die an unterschiedlichen Orten der Datenspeichervorrichtung 3 abgelegt werden. Dies ist hier durch die obere und die untere Hälfte des Speichers angedeutet.

Die Datenstruktur ist derart, dass die Datensätze jeweils den gleichen Kopf mit einer gleichen Abfolge von Grunddaten und einen Rumpf mit einem auf das jeweilige Überwachungsereignis zugeschnittenen Umfang verfügen. Dabei ermöglicht ein Link (hier durch die Pfeile angedeutet) die Verbindung zwischen Kopf und Rumpf.

Zu sehen ist, dass die Rümpfe unterschiedliche Größen aufweisen. Dies beruht in der gezeigten Variante darauf, dass zu den einzelnen Überwachungsereignissen bzw. passend zum Auftreten der Überwachungsereignisse jeweils unterschiedliche Daten bezogen auf den jeweils aktuellen Zustand des Feldgeräts 1 abgelegt werden.

Ein solcher "Snapshot" des Feldgeräts 1 bzw. der herrschenden Prozessbedingungen kann dabei je nach Art des Überwachungsereignisses unterschiedlich ausfallen. Für das Überwachungsereignis "Änderung eines Parameters" sind offensichtlich andere Daten relevant als für das Überwachungsereignis "Überschreiten eines vorgegebenen Werts für die Prozesstemperatur als sekundäre Messgröße". Daher erfolgt in der hier gezeigten Ausgestaltung eine Abspeicherung von unterschiedlich dimensionierten Daten des Rumpfs eines Datensatzes.

Für den - hier nicht dargestellten - Fall, dass alle Datensätze unabhängig vom spezifischen Überwachungsereignis die gleiche Datengröße haben sollen, werden ggf. Felder mit Platzhaltern aufgefüllt. Werden beispielweise die Änderungen von Parametern mit den gleichen Datensätzen protokolliert wie das Überschreiten eines Grenzwerts, so werden beispielsweise die Datenplätze für die Daten der geänderten Parameter beim Auftreten eines Überschreitens eines Grenzwerts durch Platzhalter aufgefüllt.

Die fünf Datensätze sind hier in der gezeigten Datenspeichervorrichtung 3 aufeinanderfolgend abgelegt. In einer alternativen Variante ist für jedes Überwachungsereignis oder zumindest jede Art von Überwachungsereignis ein eigener Bereich des Speichers der Datenspeichervorrichtung 3 vorgehalten. In einer weiteren Variante werden die Daten jeweils so abgelegt, dass alle Bereiche des Speichers ungefähr gleich oft beschrieben werden, um eine Art der Ermüdung der Abschnitte durch ein öfters auftretendes Schreiben und Überschreiben möglichst zu vermeiden.

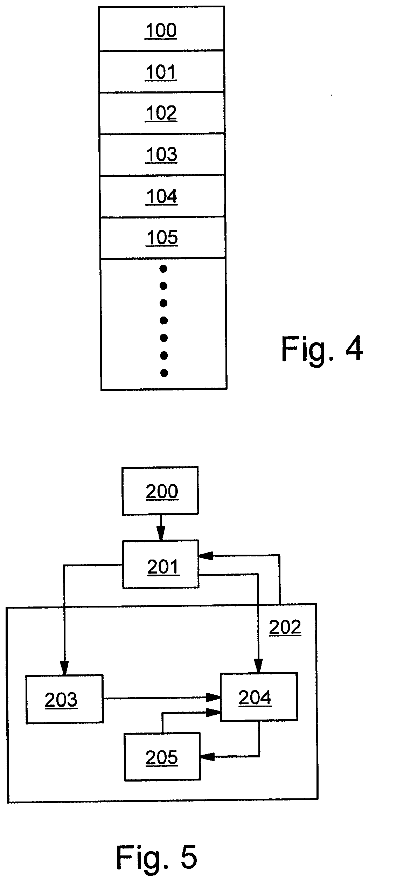

In der Fig. 4 ist ein Datensatz mit den in ihm befindlichen Daten angedeutet.

In dem obersten Block 100 wird ein Wert bzw. ein Code für das jeweilige Überwachungsereignis eingetragen.

Im Block 101 folgt ein Status-Code, der das Auftreten des Überwachungsereignisses in Relation zu dessen "Vorgeschichte" setzt. Details dazu werden mit der Fig. 5 geschildert.

Daran schließt sich in Block 102 ein Zähler an, der das Auftreten des Überwachungsereignisses erfasst. Bei sich wiederholenden Überwachungsereignissen werden auch ggf. einzelne ältere Datensätze gelöscht oder überschrieben.

Die Datenblöcke 100 bis 102 werden beispielweise als Kopf der Datensätze - wie in der Fig. 3 dargestellt - separat vom jeweiligen Rumpf abgelegt.

Daran schließen sich in den Blöcken 103, 104, 105 usw. einzelne Daten eines "Snapshots" des Feldgeräts bzw. der Prozessbedingungen an. Dabei handelt es sich beispielsweise unter anderem um den Wert der primären Messgröße, den der sekundären Messgröße, einzelne für die jeweilige Messgrößen berechnete Zwischenwerte, um relevante und insbesondere eingestellte Parameter, um die Messunsicherheit der einzelnen Werte der Messgrößen usw. Solche Daten sind insbesondere vorteilhaft für den Fall, dass als Überwachungsereignis das Überschreiten eines Intervalls durch einen Wert einer Messgröße festgelegt ist.

Bei der Protokollierung einer Änderung von Parametern sind diese Daten z. B. ein Wert, der als Code den betroffenen Parameter kennzeichnet, ein alter Wert des Parameters und der neue Wert und ggf. eine Identifizierung über die die Änderung vornehmenden Instanz in Bezug auf die Zugangsberechtigung oder in Bezug auf die jeweilige Person.

Generell werden die Daten als Momentaufnahme oder "Snapshot" abgelegt, die besonders wichtig als Rahmenbedingungen für das jeweilige Überwachungsereignis sind. Daher ist es offensichtlich, dass bei unterschiedlichen zu überwachenden und zu protokollierenden Überwachungsereignissen auch die Daten der zugeordneten Datensätze deutlich unterschiedlich sein können.

Die Fig. 5 zeigt einen möglichen Ablauf zur Quantifizierung des Auftretens von Überwachungsereignissen, für den dann im Datensatz ein entsprechender Status-Code (in der Fig. 4 im Block 101) abgelegt wird. Das Vorliegen des Überwachungsereignisses sei dabei für die weitere Betrachtung ein außergewöhnlicher Zustand, der im normalen Betrieb nicht auftreten sollte bzw. nicht auftritt.

Gestartet wird im Punkt 200 durch eine Inbetriebnahme des Feldgeräts.

Für jeden der folgenden Schritte: 201 bis 205 wird dabei in dem Datensatz jeweils ein Zähler für den Status-Code auf einen passenden Wert gesetzt.

So wird im Punkt 201 eine Initialisierung vorgenommen, wofür der Status-Code auf Null gesetzt wird. Daran schließt sich der normale Betrieb des Feldgeräts an, dessen Schritte hier in dem Block 202 angeordnet sind. Aus diesem Block 202 ist dabei auch wieder ein Rücksetzen des Feldgerätes durch eine erneute Initialisierung im Schritt 201 möglich (angedeutet durch den Pfeil zurück vom Block 202 zum Block 201).

Nach der Initialisierung im Schritt 201 folgt in dem Fall, dass das Überwachungsereignis nicht auftritt, d. h. dass ein normaler Zustand gegeben ist, der Schritt 203, der dadurch protokolliert wird, dass der Status-Code auf den Wert Eins gesetzt wird.

Zeigt sich nach der Initialisierung das Überwachungsereignis, so folgt Schritt 204, in dem der Status-Code auf den Wert Zwei gesetzt wird. Da das Überwachungsereignis eine Ausnahme vom normalen Verhalten darstellt, verschwindet vorzugsweise das Überwachungsereignis wieder. Daher wird im Schritt 205 der Status-Code auf den Wert Vier gesetzt in dem Sinne, dass das System oder das Feldgerät aus dem Zustand, den das Überwachungsereignis kennzeichnet, wieder herausgegangen ist.

Nach den Schritten 203 oder 205 können jedoch wieder die Bedingungen für das Überwachungsereignis und den Schritt 204 auftreten.

Sollte ein solcher Rückfall in das Überwachungsereignis (Weg von Schritt 204 über 205 zu 204) oder ein erstmaliges Auftreten des Überwachungsereignisses (Weg von Schritt 203 zu 204) stattfinden, so wird in einer Variante der Status-Code entsprechend erhöht, um diesen Übergangsweg zu kennzeichnen. In einer alternativen Variante wird der Status-Code in beiden Fällen auf den Wert Zwei gesetzt, jedoch durch einen Zähler für das Auftreten des Überwachungsereignisses wird festgehalten, dass gleichsam ein Rücksprung stattgefunden hat.

Insgesamt erlaubt der Status-Code, ggf. in Verbindung mit dem Zähler über die Anzahl des Auftretens des Überwachungsereignisses einen schnellen Überblick über das Verhalten des Feldgeräts bzw. über die Abläufe in dem Umfeld, in dem das Feldgerät zum Einsatz kommt.

Bei der Ablage solcher Zählern greift die Steuereinheit 4 in einer Ausgestaltung auch teilweise auf die in der Datenspeichervorrichtung 3 abgelegten Datensätze zurück, um die notwendigen Informationen über die vorhergehenden Überwachungsereignisse zu erhalten.

## Patentansprüche

1. Feldgerät (1) mit mindestens einer Messeinheit (2) zum Ermitteln mindestens einer Messgröße, mit mindestens einer als integraler Bestandteil des Feldgeräts (1) ausgestalteten Datenspeichervorrichtung (3) und mit mindestens einer als integraler Bestandteil des Feldgeräts (1) ausgestalteten Steuereinheit (4), wobei die Steuereinheit (4) in Abhängigkeit von mindestens einem vorgebbaren Überwachungsereignis mindestens einen Datensatz in der Datenspeichervorrichtung (3) ablegt und wobei die Steuereinheit (4) als Datensatz mehrere vorgebbare Daten des Feldgeräts (1) ablegt,
**dadurch gekennzeichnet,**
**dass** die Steuereinheit (4) eine Anzahl und/oder eine Größe der Daten des Datensatzes in Abhängigkeit von einer Speichergröße der Datenspeichervorrichtung (3) und/oder einer für eine Speicherung des Datensatzes zur Verfügung stehenden Speichergröße der Datenspeichervorrichtung (3) festsetzt.

2. Feldgerät (1) nach Anspruch 1, **dadurch gekennzeichnet, dass** mindestens eine Sensoreinheit (6) zum Ermitteln mindestens einer sekundären Messgröße vorgesehen ist und dass das Überwachungsereignis darin besteht, dass der von der Sensoreinheit (6) ermittelte Wert der sekundären Messgröße außerhalb eines vorgebbaren Intervalls liegt.

3. Feldgerät (1) nach Anspruch 1 oder 2, **dadurch gekennzeichnet, dass** mindestens eine Zugriffsschnittstelle (7) zur Ermöglichung eines Zugriffs auf das Feldgerät (1) vorgesehen ist und dass das Überwachungsereignis darin besteht, dass über die Zugriffsschnittstelle (7) ein Zugriff und/oder eine bestimmte Art von Zugriff auf das Feldgerät (1) stattfindet.

4. Feldgerät (1) nach einem der Ansprüche 1 bis 3, **dadurch gekennzeichnet, dass** es sich bei den vorgebbaren Daten, die die Steuereinheit (4) als Datensatz ablegt, mindestens um einen dem Überwachungsereignis zugeordneten Wert oder um eine Angabe eines Zeitpunkts oder um einen einer Abfolge eines Auftretens des Überwachungsereignisses zugeordneten Wert oder um eine Angabe über eine Betriebsdauer des Feldgeräts (1) oder um mindestens einen vom Feldgerät (1) ermittelten Wert für eine Messgröße oder um mindestens einen innerhalb des Feldgeräts (1) erzeugten und/oder berechneten Wert handelt.

5. Feldgerät (1) nach einem der Ansprüche 1 bis 4, **dadurch gekennzeichnet, dass** die Steuereinheit (4) bei einem wiederholten Auftreten eines Überwachungsereignisses eine Anzahl des Auftretens des Überwachungsereignisses als Datensatz und/oder als Teil eines Datensatzes in der Datenspeichervorrichtung (3) ablegt.

6. Feldgerät (1) nach einem der Ansprüche 1 bis 5, **dadurch gekennzeichnet, dass** mindestens eine Datenschnittstelle (7) für einen lesenden Zugriff auf die Datenspeichervorrichtung (3) vorgesehen ist und dass die Steuereinheit (4) bei einem lesenden Zugriff auf die Datenspeichervorrichtung (3) das Auftreten des lesenden Zugriffs als Datensatz und/oder als Teil eines Datensatzes in der Datenspeichervorrichtung (3) ablegt.

## Claims

1. Field device (1) with at least one measuring unit (2) for determining at least one measured variable, with at least one data storage device (3) designed as an integral component of the field device (1) and with at least one control unit (4) designed as an integral component of the field device (1), wherein the control unit (4) stores at least one data record in the data storage device (3) depending on at least one predeterminable monitoring event and wherein the control unit (4) stores a plurality of predeterminable data of the field device (1) as a data record,
**characterized in**
**that** the control unit (4) sets a number and/or a size of the data of the data record depending on a storage size of the data storage device (3) and/or a storage size of the data storage device (3) available for storing the data record.

2. Field device (1) according to claim 1, **characterized in that** at least one sensor unit (6) is provided for determining at least one secondary measured variable and that the monitoring event consists of the value of the secondary measured variable determined by the sensor unit (6) being outside a predeterminable interval.

3. Field device (1) according to claim 1 or 2, **characterized in that** at least one access interface (7) is provided to enable access to the field device (1) and that the monitoring event consists of access and/or a specific type of access to the field device (1) taking place via the access interface (7).

4. Field device (1) according to any one of claims 1 to 3, **characterized in that** the predeterminable data, which the control unit (4) stores as a data record, is at least one value assigned to the monitoring event or an indication of a point in time or a value assigned to a sequence of an occurrence of the monitoring event or an indication of an operating time of the field device (1) or at least one value determined by the field device (1) for a measured variable or at least one value generated and/or calculated within the field device (1).

5. Field device (1) according to any one of claims 1 to 4, **characterized in that** the control unit (4), in the event of a repeated occurrence of a monitoring event, stores a number of the occurrences of the monitoring event as a data record and/or as part of a data record in the data storage device (3).

6. Field device (1) according to any one of claims 1 to 5, **characterized in that** at least one data interface (7) is provided for a read access to the data storage device (3) and that the control unit (4), in the event of a read access to the data storage device (3), stores the occurrence of the read access as a data record and/or as part of a data record in the data storage device (3).

## Revendications

1. Appareil de terrain (1) comprenant au moins une unité de mesure (2) destinée à déterminer au moins une grandeur de mesure, comprenant au moins un arrangement de mémorisation de données (3) configuré en tant que partie intégrante de l'appareil de terrain (1) et comprenant au moins une unité de commande (4) configurée en tant que partie intégrante de l'appareil de terrain (1), l'unité de commande (4) déposant au moins un jeu de données dans l'arrangement de mémorisation de données (3) en fonction d'au moins un événement de surveillance pouvant être prédéfini et l'unité de commande (4) déposant, en tant que jeu de données, plusieurs données pouvant être prédéfinies de l'appareil de terrain (1),
**caractérisé en ce que** l'unité de commande (4) définit un nombre et/ou un volume des données du jeu de données en fonction d'une taille de mémoire de l'arrangement de mémorisation de données (3) et/ou d'une taille de mémoire de l'arrangement de mémorisation de données (3) disponible pour la mémorisation du jeu de données.

2. Appareil de terrain (1) selon la revendication 1, **caractérisé en ce qu'**au moins une unité de détection (6) destinée à déterminer au moins une grandeur de mesure secondaire est présente et **en ce que** l'événement de surveillance consiste **en ce que** la valeur de la grandeur de mesure secondaire déterminée par l'unité de détection (6) se trouve en-dehors d'un intervalle pouvant être prédéfini.

3. Appareil de terrain (1) selon la revendication 1 ou 2, **caractérisé en ce qu'**au moins une interface d'accès (7) destinée à rendre possible un accès à l'appareil de terrain (1) est présente et **en ce que** l'événement de surveillance consiste **en ce qu'**un accès et/ou un type déterminé d'accès à l'appareil de terrain (1) a lieu par le biais de l'interface d'accès (7).

4. Appareil de terrain (1) selon l'une des revendications 1 à 3, **caractérisé en ce que** les données pouvant être prédéfinies que l'unité de commande (4) dépose en tant que jeu de données sont au moins une valeur associée à l'événement de surveillance ou une indication d'un instant ou une valeur associée à une séquence d'une survenance de l'événement de surveillance ou une indication à propos d'une durée de fonctionnement de l'appareil de terrain (1) ou au moins une valeur déterminée par l'appareil de terrain (1) pour une grandeur de mesure ou au moins une valeur générée et/ou calculée à l'intérieur de l'appareil de terrain (1).

5. Appareil de terrain (1) selon l'une des revendications 1 à 4, **caractérisé en ce que** l'unité de commande (4), en cas de survenance répétée d'un événement de surveillance, dépose un nombre de survenances de l'événement de surveillance en tant que jeu de données et/ou en tant que partie d'un jeu de données dans l'arrangement de mémorisation de données (3) .

6. Appareil de terrain (1) selon l'une des revendications 1 à 5, **caractérisé en ce qu'**au moins une interface de données (7) pour un accès en lecture à l'arrangement de mémorisation de données (3) est présente et **en ce que** l'unité de commande (4), dans le cas d'un accès en lecture à l'arrangement de mémorisation de données (3), dépose la survenance de l'accès en lecture en tant que jeu de données et/ou en tant que partie d'un jeu de données dans l'arrangement de mémorisation de données (3).
